**Europäisches Patentamt**

**European Patent Office**

**Office européen des brevets**

(11) Veröffentlichungsnummer: **0 308 947**

**A2**

## (12) EUROPÄISCHE PATENTANMELDUNG

(21) Anmeldenummer: **88115633.5**

(22) Anmeldetag: **23.09.88**

(51) Int. Cl.4: **C12M 1/00 , C12N 1/00**

(30) Priorität: **25.09.87 DE 3732370**

(43) Veröffentlichungstag der Anmeldung:
**29.03.89 Patentblatt 89/13**

(84) Benannte Vertragsstaaten:
**AT BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Rothert, Reinhard**
**Idsteiner Strasse 86**
**D-6272 Niedernhausen/Taunus(DE)**

(54) **Verfahren zur Vermischung von lebenden Zellen oder Mikroorganismen mit einer viskosen Flüssigkeit und Mischungen, die nach diesem Verfahren hergestellt wurden.**

(57) Lebende Zellen oder Mikroorganismen werden mittels eines statischen Mischers mit einer viskosen Flüssigkeit homogen vermischt. Derartige Mischungen eignen sich unter anderem zur Mikroenkapsulierung von lebenden Zellen oder Mikroorganismen.

**EP 0 308 947 A2**

## Verfahren zur Vermischung von lebenden Zellen oder Mikroorganismen mit einer viskosen Flüssigkeit und Mischungen, die nach diesem Verfahren hergestellt wurden

Für bestimmte Zwecke kann es nötig sein, lebende Zellen oder Mikroorganismen mit einer viskosen Flüssigkeit homogen zu vermischen. Ein solcher Zweck ist z. B. die Mikroenkapsulierung von Zellen oder Mikroorganismen. Bisher wurden zur Herstellung derartiger Mischungen teilweise spezielle Ausführungen dynamischer Mischer benutzt. Die Verwendung dynamischer Mischer ist jedoch mit einigen gravierenden Nachteilen verknüpft, die zur Inaktivierung der biologischen Systeme führen können. Durch das dynamische Rührwerk werden die Zellen und Mikroorganismen insbesondere durch die auftretenden Scherkräfte physikalisch stark belastet. Die auftretenden Scherkräfte steigern sich mit der Viskosität der jeweiligen Flüssigkeit. Um die Belastung durch das Rührwerk in Grenzen zu halten, wird mit relativ geringer Intensität gerührt, was wiederum zu unvollständiger Vermischung bzw. zu relative langer Verweilzeit der biologischen Systeme in einer unphysiologischen Umgebung führt. Weiterhin kann es notwendig sein, zwecks definierter und vollständig homogener Vermischung der Systeme, im diskontinuierlichen Betrieb - mit allen bekannten Nachteilen - zu arbeiten.

In dem Bestreben, ein besseres Verfahren zur Vermischung von lebenden Zellen oder Mikroorganismen mit einer viskosen Flüssigkeit bereitzustellen, wurde nun gefunden, daß statische Mischer für diese Zwecke hervorragend geeignet sind. Dies ist überraschend, da es bei Einsatz eines statischen Mischers zur Schädigung der biologischen Systeme kommen kann, insbesondere durch den in einem statischen Mischer beim Durchströmen einer viskosen Flüssigkeit auftretenden Druckabfall.

Erfindungsgegenstand ist demzufolge ein Verfahren zur homogenen Verteilung von lebenden Zellen oder Mikroorganismen in einer viskosen Flüssigkeit durch Vermischen der lebenden Zellen oder Mikroorganismen mit der viskosen Flüssigkeit, das dadurch gekennzeichnet ist, daß man zur Vermischung einen statischen Mischer benutzt. Das erfindungsgemäße Mischverfahren hat gegenüber der Benutzung dynamischer Mischer folgende Vorteile:
- zur vollständigen schonenden Vermischung werden kürzere Verweilzeiten benötigt (vgl. Beispiel 1)
- die Scherbelastung der biologischen Systeme ist geringer
- ein Energieeintrag über Rührwerke etc. findet nicht statt
- die Apparatur enthält keine beweglichen Teile, die eventuell einer Schmierung und/oder einer Abdichtung bedürfen
- ein Betrieb in geschlossenem (ggfs. keimfreiem) System ist leichter realisierbar.

Mit dem erfindungsgemäßen Verfahren lassen sich beliebige lebende Zellen oder Mikroorganismen mit beliebigen viskosen Flüssigkeiten vermischen. Als bevorzugte Beispiele für lebende Zellen und Mikroorganismen seien z.B. genannt Hybridomazellen, wie z.B. antikörperproduzierende Zellen, Pflanzenzellen, wie z.B. Nutzpflanzenzellen, Bakterienzellen wie z.B. E.coli und Milchsäurebakterien, Streptomyceten, Pilze wie z.B. Penicillinium und Aspergillus und Hefen wie z.B. Bäckerhefe.

Besonders bevorzugt sind antikörperproduzierende Zellen, Nutzpflanzenzellen, E. coli und Bäkkerhefe, insbesondere antikörperproduzierende Zellen und Nutzpflanzenzellen.

Die viskose Flüssigkeit besitzt vorzugsweise eine Viskosität von ca. 0,002 - 10 Pa.s, besonders bevorzugt von ca. 0,005 - 1 Pa.s, insbesondere von ca. 0,01 - 0,6 Pa.s. Die Vermischung der lebenden Zellen oder Mikroorganismen kann mit beliebigen Flüssigkeiten, die nicht zur Zerstörung der Systeme führen, erfolgen. Besondere Bedeutung besitzt das Verfahren für die Vermischung der genannten biologischen Systeme mit Polymerlösungen.

Die räumliche Gestaltung der für das erfindungsgemäße Verfahren geeigneten Mischelemente ist an sich beliebig. Vorzuziehen ist eine räumliche Gestaltung der Mischelemente in Form eines Gerüsts ineinandergreifender, sich kreuzender Stege wie z. B. ein [R]Sulzer statischer Mischer SMX der Firma Gebrüder Sulzer Aktiengesellschaft, CH-8401 Winterthur, Schweiz. Es lassen sich beliebig viele Mischelemente in gemeinsamen oder getrennten Gehäusen parallelschalten. Die Anzahl hintereinandergeschalteter Mischelemente ist im Prinzip ebenfalls beliebig, zu bevorzugen ist jedoch eine Anzahl von 1 - 10, besonders bevorzugt von 2 - 6, insbesondere von 4 Mischelementen pro statischem Mischer.

Die Geschwindigkeit, mit der die zu mischenden Substanzen den statischen Mischer durchströmen, kann über einen weiten Bereich variiert werden. Zu bevorzugen ist eine Strömungsgeschwindigkeit, bei der - zur Schonung der biologischen Systeme - die Strömung weitgehend laminar verläuft; diese Geschwindigkeit ist wiederum abhängig von der räumlichen Gestaltung und den Abmessungen des statischen Mischers.

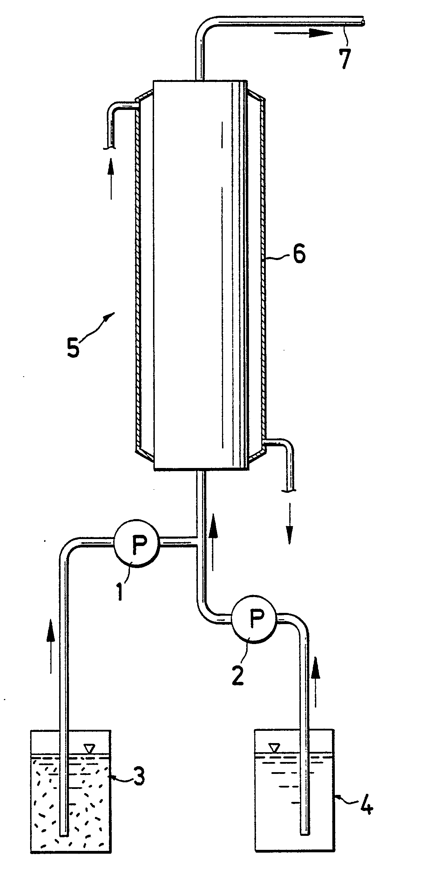

Eine Vorrichtung, mit der das erfindungsgemäße Verfahren durchführbar ist, kann z. B. den in der Figur schematisch dargestellten Aufbau haben. Mit den Pumpen (1) und (2) werden die lebenden Zellen oder Mikroorganismen, die in Vorratsgefäß (3) in einer Lösemittelsuspension vorliegen, und die viskose Flüssigkeit aus Vorratsgefäß (4) über die Zuleitungen in den statischen Mischer gepumpt. Das Lösemittel zur Suspension ist beliebig, vorzuziehen ist Wasser, ggfs. mit Zusätzen, die für die Zellen oder Mikroorganismen vorteilhaft sind, wie z. B. Nährstoffzusätze etc. Es ist sinnvoll, die gesamte Anlage oder auch nur das bzw. die Mischelemente (5) wie. in der Figur dargestellt, mit einer Temperiereinrichtung (6) zu versehen. Nach dem Durchströmen des Mischers kann die erhaltene Mischung auf beliebige Weise der weiteren Verwendung zugeführt werden.

Durch die nachfolgenden Ausführungsbeispiele soll die Erfindung näher erläutert werden.

Beispiele

Beispiel 1

30 ml einer 3 gew.-%igen λ-dCarrageen Lsg. (Hersteller Sigma Chemie GmbH, München) wird über eine Schlauchpumpe dem statischen Mischer mit einer Pumpleistung von 850 μl/min zugeführt. 30 ml Medium (Dulbeccos Medium mit fötalem Kälberserum wird mit einer Pumpleistung von 850 μl/min und einer zweiten Schlauchpumpe dem Mischer zugeführt. Der eingesetzte statische Mischer besteht aus 4 Mischelementen (SMX-Mischer DN4 der Fa. Gebrüder Sulzer AG). Die so hergestellte Suspension wird im Spektrophotometer bei 750 nm analysiert und auf Homogenität untersucht.

Die gemessenen Werte sind mit denen aus anderen Mischsystemen vergleichbar, bei nur ca. 20 sec Verweilzeit im Mischsystem gegenüber mehreren Minuten bei dynamischer Mischung.

Beispiel 2

30 ml einer 3 gew.-%igen λ-Carrageen Lsg. wird mit Hilfe einer Schlauchpumpe mit einer Pumpleistung von 850 μl/min zugeführt. 30 ml Medium mit einer Zelldichte von $10^5$ Zellen/ml wird dem Mischer mit einer zweiten Schlauchpumpe mit einer Pumpleistung von 850 μl/min zugeführt.

Der eingesetzte Mischer besteht aus 4 Mischelementen (SMX-Mischer DN4 der Firma Gebrüder Sulzer AG). Die so hergestellte Suspension wird über eine Vertropfungseinheit zu Mikrotropfen vertropft, die mit einer Polybase (Copolymerisat aus Vinylimidazoliummethochlorid und Vinylpyrrolidon) in Kontakt gebracht werden. Die entstehenden Kapseln enthalten ca. 30 Zellen und sind einheitlich. Mikroskopische Untersuchungen sowie Langzeitbeobachtungen zeigen keine nachweisbare Schädigung der Zellen.

**Ansprüche**

1. Verfahren zur homogenen Verteilung von lebenden Zellen oder Mikroorganismen in einer viskosen Flüssigkeit durch Vermischen der lebenden Zellen oder Mikroorganismen mit der viskosen Flüssigkeit, dadurch gekennzeichnet, daß man zur Vermischung einen statischen Mischer benutzt.

2. Verfahren nach Anspruch 1, dadurch gekennzeichnet, daß die viskose Flüssigkeit eine Viskosität von ca. 0,002 bis 10 Pa.s, vorzugsweise von ca. 0,005 bis 1 Pa.s, insbesondere von ca. 0,01, bis 0,6 Pa.s besitzt.

3. Verfahren nach Anspruch 1 oder 2, dadurch gekennzeichnet, daß die viskose Flüssigkeit eine Polymerlösung ist.

4. Verfahren nach einem oder mehreren der Ansprüche 1 bis 3, dadurch gekennzeichnet, daß der statische Mischer 1 bis 10, vorzugsweise 2 bis 6, insbesondere 4 Mischelemente aufweist.

5. Verfahren nach einem oder mehreren der Ansprüche 1 bis 4, dadurch gekennzeichnet, daß die Vermischung bei laminarer Strömung erfolgt.

6. Mischungen von lebenden Zellen oder Mikroorganismen mit einer viskosen Lösung, dadurch gekennzeichnet, daß die Mischung mit einem Verfahren nach einem oder mehreren der Ansprüche 1 bis 5 hergestellt wurde.